# EUROPÄISCHE PATENTSCHRIFT

(11) **EP 0 992 489 B1**
(45) Veröffentlichungstag und Bekanntmachung des Hinweises auf die Patenterteilung: **18.05.2005**
(21) Anmeldenummer: 99119409.3
(22) Anmeldetag: 30.09.1999
(51) Int. Cl.: C07C 235/28, C07C 235/10, C11D 1/52, A61K 7/08, A61K 47/18

(54) **Betaine**
Betaines
Betaines

(30) Priorität: 09.10.1998 DE 19846537
(43) Veröffentlichungstag der Anmeldung: 12.04.2000
(73) Patentinhaber: Cognis Deutschland GmbH & Co. KG, 40589 Düsseldorf (DE)
(72) Erfinder: Bonastre Nuria, Gilabert, Dr., 08210 Barbera del Vall (ES); Conesa Amela, Christina, 08042 Barcelon (ES); Pi Subirana, Rafael, Dr., 08400 Granollers (ES); Bigorra Llosas, Joaquin, Dr., 08203 Sabadell (ES)

(56) Entgegenhaltungen:
- DE-A- 19 647 636
- FR-A- 2 639 635
- HEIN, H.: "Surface active derivatives of ricinoleic acid" FETTE, SEIFEN, ANSTRICHM. (1985), 87(7), 283-9 , XP002128788
- FORMO M.W.: 'Bailey's industrial oil and fat products', WILEY-INTERSCIENCE, NEW YORK

## Beschreibung

### Gebiet der Erfindung

Die Erfindung befindet sich auf dem Gebiet der Kosmetik und betrifft neue amphotere Verbindungen, Verfahren zu ihrer Herstellung sowie deren Verwendung zur Herstellung von kosmetischen und pharmazeutischen Zubereitungen.

### Stand der Technik

Betaine stellen bekannte Tenside dar, die überwiegend durch Carboxyalkylierung, vorzugsweise Carb-oxymethylierung von aminischen Verbindungen hergestellt werden. Vorzugsweise werden die Aus-gangsstoffe mit Halogencarbonsäuren oder deren Salzen, insbesondere mit Natriumchloracetat kon-densiert, wobei pro Mol Betain ein Mol Salz gebildet wird. Ferner ist auch die Anlagerung von unge-sättigten Carbonsäuren, wie beispielsweise Acrylsäure möglich. Zur Nomenklatur und insbesondere zur Unterscheidung zwischen Betainen und "echten" Amphotensiden sei auf den Beitrag von U.Ploog in **Seifen-Öle-Fette-Wachse,** **108**, **373 (1982)** verwiesen. Weitere Übersichten zu diesem Thema finden sich beispielsweise von A.O'Lennick et al. in **HAPPI, Nov. 70 (1986),** S.Holzman et al. in Tens. **Surf.Det.** **23**, **309 (1986),** R.Bilbo et al. in **Soap Cosm.Chem.Spec., Apr. 46 (1990)** und P.Ellis et al. in **Euro Cosm.** **1**, **14 (1994).** Eine weitere Übersicht über oberflächenaktive Derivate von Rizinolsäure, insbesondere Ethoxylierungs- und Sulfatierungsprodukte, findet sind bei H.Hein in Fette, Seifen, Anstrichm. (1985), 87(7), 283-9.

Amphotere Tenside, wie das bekannte Cocamidopropyl Betain, stellen gangige Einsatzprodukte für die Herstellung von kosmetischen Zubereitungen, aber auch von manuellen Geschirrspülmitteln dar, da sie nicht nur vorteilhafte reinigende und emulgierende Eigenschaften besitzen, sondern dazu sehr gut hautverträglich sind und die dermatologische Verträglichkeit von Tensidmischungen synergistisch verbessern können. Ein bislang ungelöstes Problem besteht jedoch darin, daß sich die bekannten Betaine in kaltem Wasser eher schlecht lösen und es einigen Aufwands bedarf, die meist stark getrübten Produkte zu klaren Endformulierungen zu verarbeiten. Des weiteren sind die anwendungs-technischen Eigenschaften der Produkte nicht immer zufriedenstellend.

Demzufolge hat die Aufgabe der vorliegenden Erfindung darin bestanden, neue Betaine zur Verfügung zu stellen, die frei von den geschilderten Nachteilen sind, d.h. verbesserte anwendungstechnische Ei-genschaften aufweisen und sich insbesondere auch in kaltem Wasser rasch und trübungstrei lösen.

### Beschreibung der Erfindung

Gegenstand der Erfindung sind Betaine der Formel **(I)** 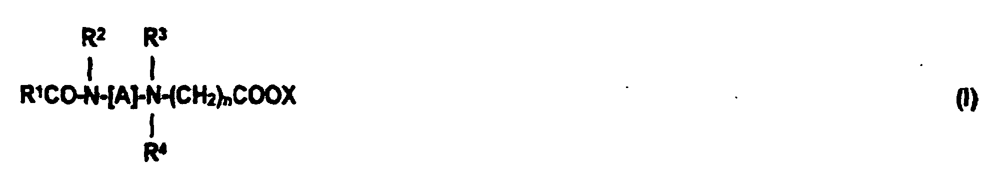 in der R¹CO für einen gesättigten und/oder ungesättigten, ethoxylierten Hydroxyacylrest mit 16 bis 22, vorzugsweise 18 Kohlenstoffatomen sowie 1 bis 50 Oxyethyteneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, n für Zahlen von 1 bis 3 und X für Wasserstoff, ein Alkali- oder Erdalkalimetall oder Ammonium steht;

Überraschenderweise wurde gefunden, daß die Anlagerung von Ethylenoxid an die OH-Gruppe von Ricinolsäure bzw. 12-Hydroxystearinsäure zu Betainen führt, die nicht nur verbesserte anwendungs-technische Eigenschaften und besondere hautkosmetische Verträglichkeit aufweisen, sondern sich auch in kaltem Wasser klar lösen und dann zu trübungsfreien Endformulierungen weiterverarbeitet werden können.

### Herstellverfahren

Ein weiterer Gegenstand der Erfindung betrifft ein Verfahren zur Herstellung von Betainen der Formel **(I)** 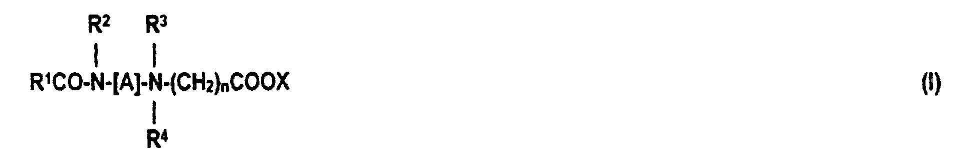 in der R¹CO für einen gesättigten und/oder ungesättigten, ethoxylierten Hydroxyacylrest mit 16 bis 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, n für Zahlen von 1 bis 3 und X für Wasserstoff, ein Alkali- oder Erdalkalimetall oder Ammonium steht, bei dem man
(a) Ricinusöl und/oder gehärtetes Ricinusöl mit - bezogen auf die Hydroxylgruppen im Acylrest - 1 bis 50 Mol Ethylenoxid ethoxyliert,
(b) die resultierende Ethoxylate mit Diaminen der Formel (II) wie unten definiert umsetzt und
(c) die resultierenden Amidoamine mit Halogenalkansäuren der Formel (III) wie unten definiert betainisiert.

Alternativ ist es auch möglich, zunächst das Triglycerid in das Amidoamin zu überführen und dieses dann zu ethoxylieren, ehe anschließend die Kondensation mit der Halogenalkansäure erfolgt. Dementsprechend betrifft ein weiterer Gegenstand der Erfindung ein Verfahren zur Herstellung von Betainen der oben genannten Formel (**I**), bei dem man
(a) Ricinusöl und/oder gehärtetes Ricinusöl mit Diaminen der Formel (II) wie unten definiert umsetzt,
(b) die resultierende Amidoamine mit - bezogen auf die Hydroxylgruppen im Acylrest - 1 bis 50 Mol Ethylenoxid ethoxyliert und
(c) die resultierenden ethoxylierten Amidoamine mit Halogenalkansäuren der Formel (III) wie unten definert betainisiert.

Schließlich ist es auch möglich, zunächst aus dem Triglycerid das Amidoamin und aus diesem das Betain herzustellen, ehe dann abschließend Ethylenoxid an die Hydroxylgruppe des Acylrestes angelagert wird. Dementsprechend betrifft ein weiterer Gegenstand der Erfindung ein drittes Verfahren zur Herstellung von Betainen der Formel (**I**), bei dem man
(a) Ricinusöl und/oder gehärtetes Ricinusöl mit Diaminen der Formel (II) wie unten definiert umsetzt,
(b) die resultierende Amidoamine mit Halogenalkansäuren der Formel (III) wie unten definiert betainisiert, und
(c) die resultierenden Betaine mit - bezogen auf die Hydroxylgruppen im Acylrest - 1 bis 50 Mol Ethylenoxid ethoxyliert.

Wegen der Anwesenheit von Wasser bei der abschließenden Ethoxylierung der Betaine kann es zur Bildung von größeren Mengen Polyethylenglycol kommen, was für eine Reihe von Anwendungen unerwünscht ist.

### Ethoxylierung

Wie oben erläutert kann die Ethoxylierung auch auf der Stufe der Amidoamine oder der Betaine durchgeführt werden. Vorzugsweise legt man jedoch die Triglyceride zusammen mit 0,5 bis 2,5 Gew.-% Katalysator (z.B. Natriummethylat oder calcinierter Hydrotalcit) in einen Rührautoklaven vor, evakuiert und gibt bei Temperaturen im Bereich von 120 bis 180 °C innerhalb von 1 bis 2 h die gewünschte Menge Ethylenoxid, vorzugsweise 5 bis 10 Mol Ethylenoxid - bezogen auf die Hydroxylgruppen im Acylrest - auf, wobei der autogene Druck bis auf 5 bar ansteigen kann. Anschließend läßt man noch etwa 30 min nachreagieren, kühlt den Reaktor ab, entspannt und neutralisiert den basischen Katalysator z.B. durch Zugabe von Milchsäure oder Phosphorsäure. Gegebenenfalls werden unlösliche Katalysatoren über eine Filterpresse abgetrennt.

### Amidierung

Die Amidierung der Triglyceride kann in an sich bekannter Weise durchgeführt werden, wobei Diamine der Formel (**II**) zum Einsatz gelangen, 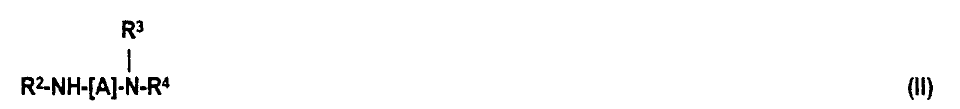 in der A für einen linearen oder verzweigten Alkylenrest mit 1 bis 6 Kohlenstoffatomen und R², R³ und R⁴ unabhängig voneinander für Wasserstoff oder eine Alkylgruppe mit 1 bis 4 Kohlenstoffatomen stehen. Neben Propylendiamin, Butylendiamin, Pentylendiamin und Hexylendiamin wird vorzugsweise Dimethylaminopropylamin (DAPA) eingesetzt. Dazu empfiehlt es sich, das Diamin - bezogen auf die für die Amidierung zur Verfügung stehenden Carboxylgruppen im Triglycerid - im geringen stöchiome-trischen Überschuß, also im molaren Verhältnis 1: 1,05 bis 1 : 1,2 einzusetzen. Die Amidierung kann in Gegenwart von für diesen Zweck üblichen alkalischen Katalysatoren, wie z.B. Natriumhydroxid, Kaliumhydroxid oder Natriummethylat durchgeführt werden; die Katalysatorkonzentration liegt in der Regel bei 0,1 bis 2 Gew.-% - bezogen auf die Einsatzstoffe. Die Mitverwendung von geringen Mengen Alkaliborhydraten oder unterphosphoriger Säure als Co-Katalysatoren empfiehlt sich, wenn es darum geht, möglichst hellfarbige Produkte herzustellen. Die Reaktionstemperatur beträgt 100 bis 150 und vorzugsweise 120 bis 140°C. Falls gewünscht, kann unter Stickstoffabdeckung gearbeitet werden. Nach Abschluß der Amidierung kann nicht umgesetztes Diamin, gegebenenfalls zusammen mit dem freigesetzten Glycerin, im Feinvakuum abdestilliert werden. Wie schon eingangs erläutert, kann die Amidierung sowohl mit dem ethoxylierten als auch dem nicht ethoxylierten Triglycerid durchgeführt werden.

### Betainisierung

Zur Überführung der vorzugsweise bereits ethoxylierten Amidoamine in die Betaine erfolgt eine Kondensation der Aminofunktion mit einer Halogencarbonsäure der Formel **(III)** eingesetzt,

**Hal-(CH**_{**2**}**)**_{**n**}**COOX** **(III)**

in der Hal für Halogen, n für Zahlen von 1 bis 3 und X für Wasserstoff, ein Alkali- oder Erdalkalimetall oder Ammonium steht. Aus Gründen der Verfügbarkeit ist hier der Einsatz von Natriumchloracetat be-vorzugt, d.h. anstelle einer Halogenalkansäure wird gleich ihr Salz eingesetzt, so daß bei der Konden-sation als Nebenprodukt ebenfalls ein Salz, vorzugsweise Kochsalz anfällt. Wie schon die Ethoxylierung und die Amidierung erfolgt auch die Betainisierung in an sich bekannter Weise, d.h. das Amidoamin wird in wäßriger Lösung mit der Halogenalkansäure bzw. deren Salz bei Temperaturen im Bereich von 70 bis 100°C kondensiert. Die Reaktionspartner werden dabei in annähernd stöchiometrischem Verhältnis, d.h. 1 : 0,95 bis 1 : 1,05 eingesetzt. Es empfiehlt sich, während der Kondensation den pH-Wert durch Zudosieren einer wäßrigen Base im alkalischen Bereich zu halten und erst das Endprodukt durch Zugabe von Mineralsäure leicht sauer einzustellen.

### Gewerbliche Anwendbarkeit

Die neuen Betaine weisen ausgezeichnete reinigende und emulgierende Eigenschaften auf, sind auch in kaltem Wasser klarlöslich und lassen sich zu trübungsfreien Endprodukten formulieren. Ein weiterer Gegenstand der Erfindung betrifft daher die Verwendung der erfindungsgemäßen Betaine zur Her-stellung von kosmetischen- und/oder pharmazeutischen Zubereitungen, in denen sie in Mengen von 0,5 bis 50, vorzugsweise 1 bis 20 und insbesondere 5 bis 15 Gew.-% - bezogen auf die Mittel - enthalten sein können.

### Kosmetische und/oder pharmazeutische Zubereitungen

Die die neuen Betaine enthaltenden Zubereitungen, wie beispielsweise Haarshampoos, Haarlotionen, Schaumbäder, Cremes, Lotionen, Salben und dergleichen, können ferner als weitere Hilfs- und Zusatz-stoffe milde Tenside, Ölkörper, Emulgatoren, Überfettungsmittel, Perlglanzwachse, Stabilisatoren, Kon-sistenzgeber, Verdickungsmittel, Polymere, Siliconverbindungen, biogene Wirkstoffe, Deowirkstoffe, Antischuppenmittel, Filmbildner, Konservierungsmittel, Hydrotrope, Solubilisatoren, UV-Lichtschutz-faktoren, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpoly-glycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoe-säure, Ester von C₂-C₁₂-Dicarbonsäuren mit linearen oder verzweigten Alkoholen mit 1 bis 22 Kohlen-stoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalko-holcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkyl-ether mit 6 bis 22 Kohlenstoffatomen pro Alkylgruppe, Ringöffnungsprodukte von epoxidierten Fett-säureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologen-gemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosac-chariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stea-rinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe min-destens 24 Kohlenstoffatome aufweisen, speziell Lauron und Distearylether; Fettsäuren wie Stea-rinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxy-fettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologen-verteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhält-lich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Poly-glycolen und Aminen, quaternierte Kollagenpolypeptide, wie beispielsweise Lauryldimonium hydroxy-propyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethyl-aminohydroxypropyldiethylentriamin (Cartaretine®/Sandoz), Copolymere der Acrylsäure mit Dime-thyldiallylammoniumchlorid (Merquat® 550/Chemviron), Polyaminopolyamide, wie z.B. beschrieben in der **FR-A 2252840** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/ Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrroli-don/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vor-liegen können. Eine detaillierte Übersicht über geeignete flüchtige Silicone findet sich zudem von Todd et al. in **Cosm.Toil.** **91**, **27 (1976).**

Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säu-ren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc.Cosm.Chem.** **24**, **281 (1973)**]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol.** **26**, **531 (1975)**]. Neben den Chlorhydraten können auch Aluminiumhydroxylactate sowie saure Aluminium/Zirko-niumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopro-pylcitrat, Tributylcitrat und insbesondere Triethylcitrat (Hydagen® CAT, Henkel KGaA, Düssel-dorf/FRG). Die Stoffe inhibieren die Enzymaktivität und reduzieren dadurch die Geruchsbildung. Wahr-scheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copoly-merisate, Polymere der Acrylsäure-reihe, quaternäre Cellulose-Derivate, Kollagen, Hyaluronsäure bzw. deren Salze und ähnliche Verbin-dungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil.** **108**, **95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylben-zyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ethylhexylester (Oc-tocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Tria-zon, wie in der **EP-A1 0818450** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsul-fonsäure und 2-Methyl-5-(2-oxo-3-bomyliden)sulfonsäure und deren Salze.

Als typische UV-A-Filter kommen insbesondere Derivate des Benzoylmethans in Frage, wie beispiels-weise 1-(4'-tert.Butylphenyl)-3-(4'-methoxyphenyl)propan-1,3-dion, 4-tert.-Butyl-4'methoxydibenzoyl-methan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metall-oxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphä-rischen Gestalt abweichende Form besitzen. Weitere geeignete UV-Lichtschutzfilter sind der Übersicht von P.Finkel in **SÖFW-Joumal 122, 543 (1996)** zu entnehmen.

Neben den beiden vorgenannten Gruppen primärer Lichtschutzstoffe können auch sekundäre Licht-schutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearyl-thiodipropionat, Thiodipropionsäure und deren Derivate (Ester, Ether, Peptide, Lipide, Nukleotide, Nuk-leoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phy-tinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fett-säuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Deri-vate, α-Glycosylrutin, Ferulasäure, Furfurylidenglucitol, Carnosin, Butylhydroxytoluol, Butylhydroxy-anisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vor-zugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufge-führten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Iso-methylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwen-det, die gemeinsam eine ansprechende Duftnote erzeugen. Auch ätherische Öle geringerer Flüch-tigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel**" **der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Herstellbeispiel H1.** In einem 2-I-Dreihalskolben mit Rührer, Rückflußkühler und Destillationsaufsatz wurden 1084 g (0,86 mol) eines Kondensationsproduktes von 8 Mol Ethylenoxid an Ricinusöl zusammen mit 0,45 g Natriumborhydrat, 342 g (3,35 mol) Dimethylaminpropylamin (DAPA) und 28 g einer 33 Gew.-%igen Lösung von Natriummethylat in Methanol vorgelegt. Die Mischung wurde über 2 h auf 130°C erhitzt und anschließend nicht umgesetztes DAPA destillativ entfernt (150°C, 40 mbar). Das resultierende Amidoamin wies einen Estergehalt von weniger als 0,5 Gew.-% auf. 600 g (1,3 mol) des Amidoamins wurden in eine zweite Rühr- und Destillationsapparatur überführt und mit 154 g (1,32 mol) Natriumchloracetat in 555 g Wasser versetzt. Die Mischung wies einen pH-Wert von etwa 8 auf und wurde 4 h bei 80 bis 90°C gerührt, wobei der pH-Wert durch kontinuierliches Nachdosieren von 25 Gew.-%iger Natriumhydroxidlösung im alkalischen Bereich gehalten wurde. Das Endprodukt wurde mit verdünnter Salzsäure auf einen pH-Wert von 5,4 eingestellt. Die leicht gelblich gefärbte Flüssigkeit wies einen Trockenrückstand von 57,6 % und einen Kochsalzgehalt von 6,4 Gew.-% auf.

**Herstellbeispiel H2.** Analog Beispiel H1 wurden zunächst 970 g (0,86 mol) Ricinusöl+5EO mit 342 g DAPA zum Amidoamin umgesetzt. 428 g (1,3 mol) des Amidoamins wurden anschließend mit 154 g (1,32) Natriumchloracetat betainisiert. Die leicht gelblich gefärbte Flüssigkeit wies einen Trockenrück-stand von 55,2 % und einen Kochsalzgehalt von 6,1 Gew.-% auf.

**Herstellbeispiel H3.** Analog Beispiel H1 wurden zunächst 1159 g (0,86 mol) Ricinusöl+10EO mit 342 g DAPA zum Amidoamin umgesetzt. 714 g (1,3 mol) des Amidoamins wurden anschließend mit 154 g (1,32) Natriumchloracetat betainisiert. Die leicht gelblich gefärbte Flüssigkeit wies einen Trockenrück-stand von 56,1 % und einen Kochsalzgehalt von 6,2 Gew.-% auf.

**Herstellbeispiel H4.** Analog Beispiel H1 wurden zunächst 1080 g (0,86 mol) hydriertes Ricinusöl+8EO mit 342 g DAPA zum Amidoamin umgesetzt. 600 g (1,3 mol) des Amidoamins wurden anschließend mit 154 g (1,32) Natriumchloracetat betainisiert. Das leicht gelblich gefärbte Produkt wies einen Trocken-rückstand von 55,0 % und einen Kochsalzgehalt von 5,9 Gew.-% auf.

Die nachfolgende Tabelle 1 zeigt eine Reihe von Beispielen zur Formulierung der erfindungsgemäßen Betaine in kosmetischen Zubereitungen.

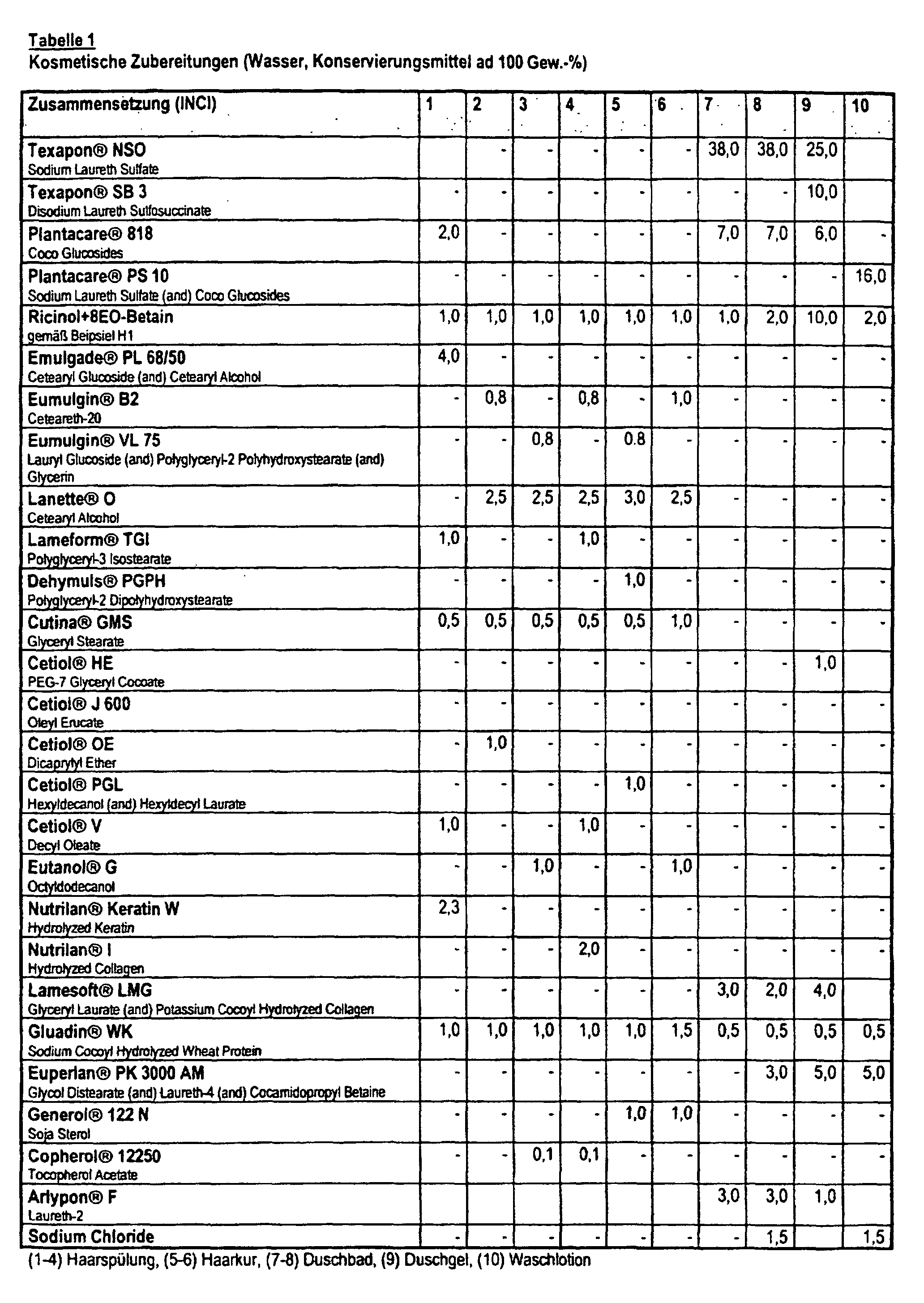

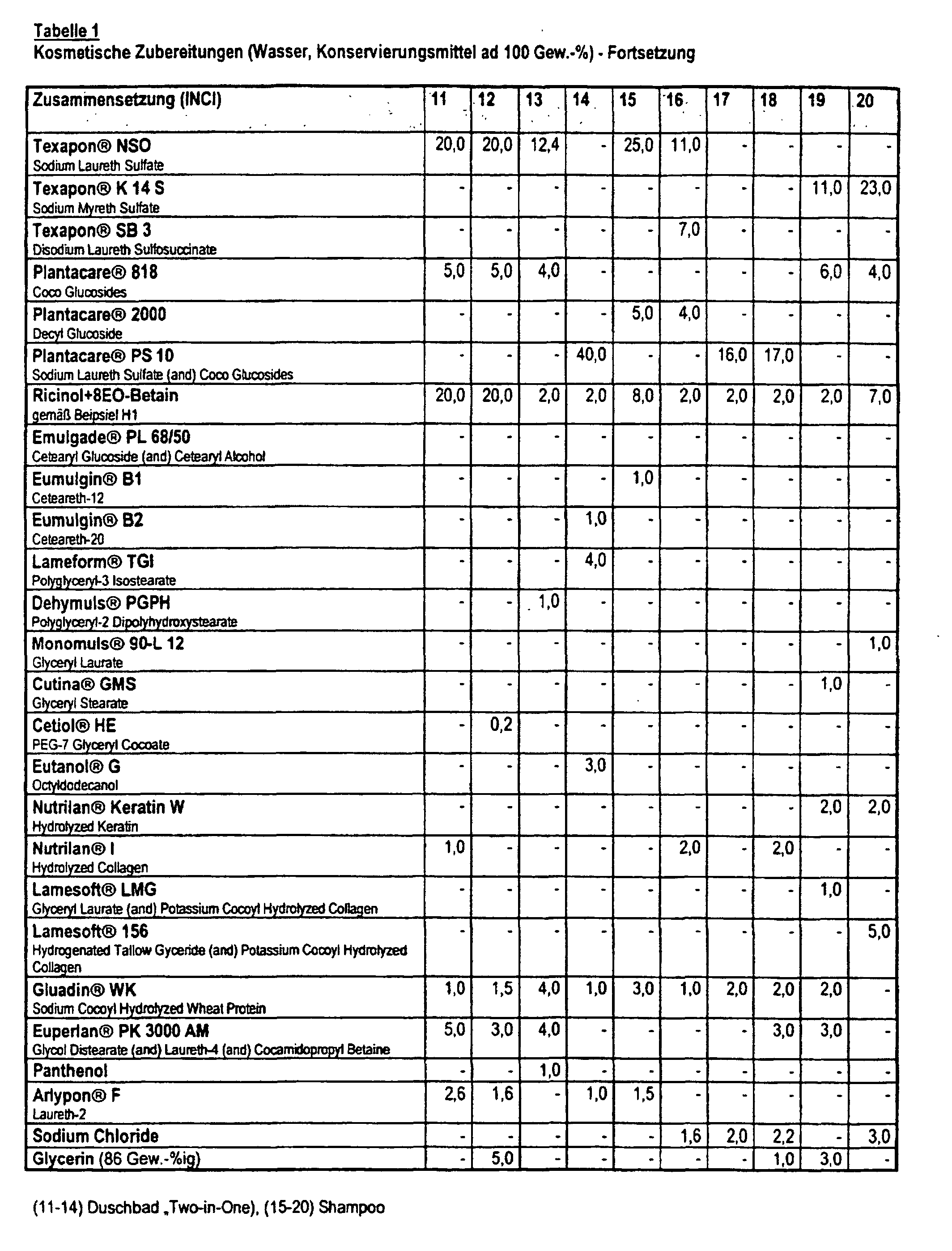

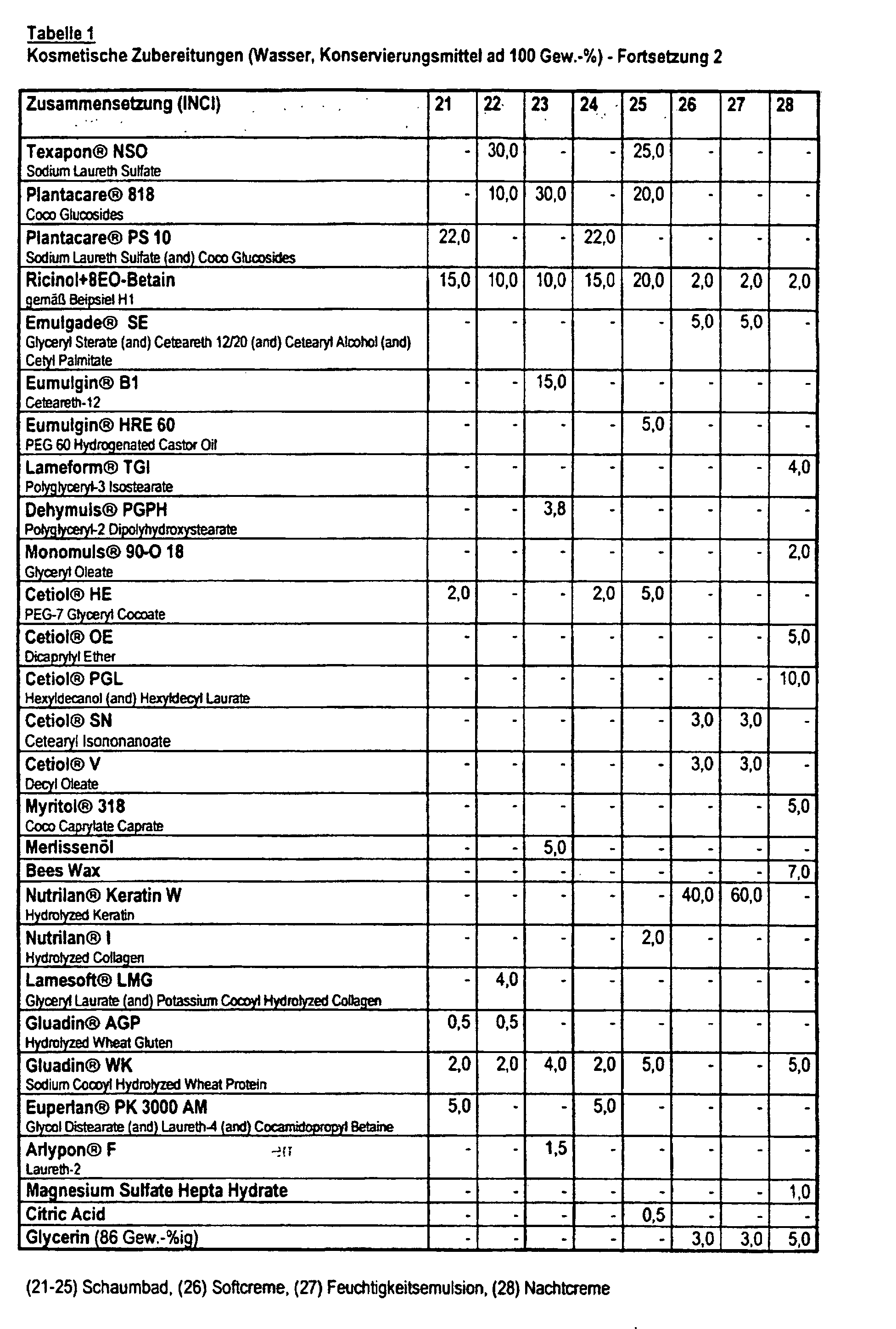

## Patentansprüche

1. Betaine der Formel **(I)**, 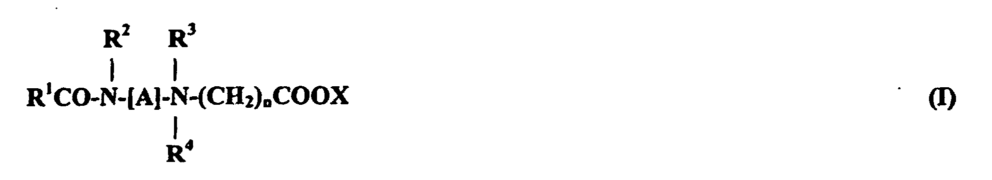 in der R¹CO für einen gesättigten und/oder ungesättigten, ethoxylierten Hydroxyacylrest mit 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, n für Zahlen von 1 bis 3 und X für Wasserstoff, ein Alkali- oder Erdalkalimetall oder Ammonium steht.

2. Verfahren zur Herstellung von Betainen der Formel **(I)**, 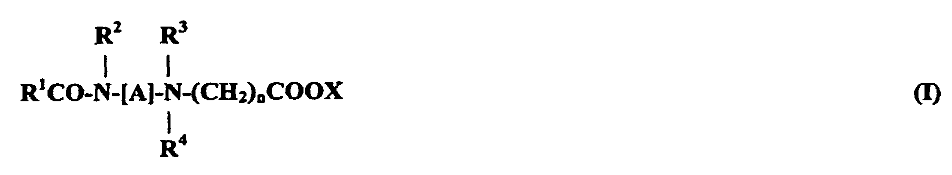 in der R¹CO für einen gesättigten und/oder ungesättigten, ethoxylierten Hydroxyacylrest mit 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, n für Zahlen von 1 bis 3 und X für Wasserstoff, ein Alkali- oder Erdalkalimetall oder Ammonium steht; bei dem man
(a) Ricinusöl und/oder gehärtetes Ricinusöl mit - bezogen auf die Hydroxylgruppen im Acylrest - 1 bis 50 Mol Ethylenoxid ethoxyliert,
(b) die resultierende Ethoxylate mit Diaminen der Formel **(II)** umsetzt, 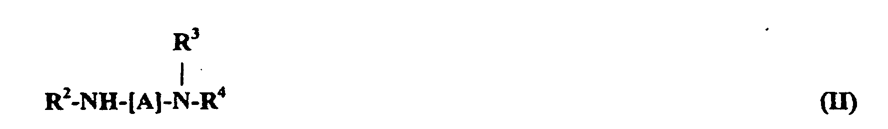 in der A für einen linearen oder verzweigten Alkylenrest mit 1 bis 6 Kohlenstoffatomen und R², R³ und R⁴ unabhängig voneinander für Wasserstoff oder eine Alkylgruppe mit 1 bis 4 Kohlenstoffatomen stehen, und
(c) die resultierenden Amidoamine mit Halogenalkansäuren der Formel **(III)** betainisiert,
**Hal-(CH**_{**2**}**)**_{**n**}**COOX** **(III)**
in der Hal für Halogen, n für Zahlen von 1 bis 3 und X für Wasserstoff, ein Alkali- oder Erd-alkalimetall oder Ammonium steht.

3. Verfahren zur Herstellung von Betainen der Formel **(I)**, 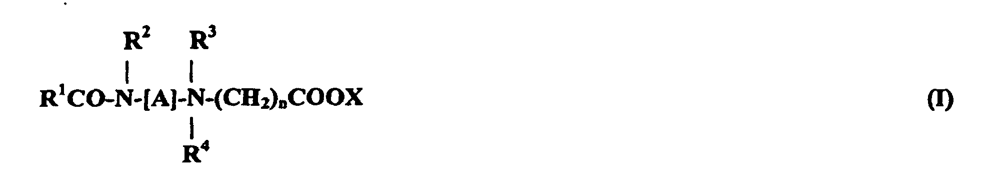 in der R¹CO für einen gesättigten und/oder ungesättigten, ethoxylierten Hydroxyacylrest mit 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, n für Zahlen von 1 bis 3 und X für Wasserstoff, ein Alkali- oder Erdalkalimetall oder Ammonium steht, bei dem man
(a) Ricinusöl und/oder gehärtetes Ricinusöl mit Diaminen der Formel **(II)** umsetzt, 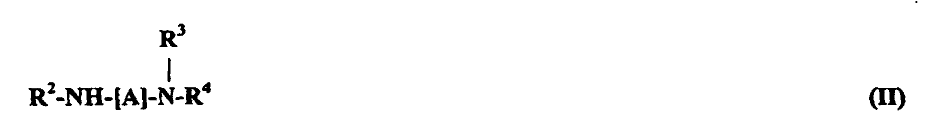 in der A für einen linearen oder verzweigten Alkylenrest mit 1 bis 6 Kohlenstoffatomen und R², R³ und R⁴ unabhängig voneinander für Wasserstoff oder eine Alkylgruppe mit 1 bis 4 Kohlenstoffatomen stehen,
(b) die resultierende Amidoamine mit - bezogen auf die Hydroxylgruppen im Acylrest - 1 bis 50 Mol Ethylenoxid ethoxyliert und
(c) die resultierenden ethoxylierten Amidoamine mit Halogenalkansäuren der Formel (III) betainisiert,
**Hal-(CH**_{**2**}**)**_{**n**}**COOX** **(III)**
in der Hal für Halogen, n für Zahlen von 1 bis 3 und X für Wasserstoff, ein Alkali- oder Erd-alkalimetall oder Ammonium steht.

4. Verfahren zur Herstellung von Betainen der Formel **(I)**, 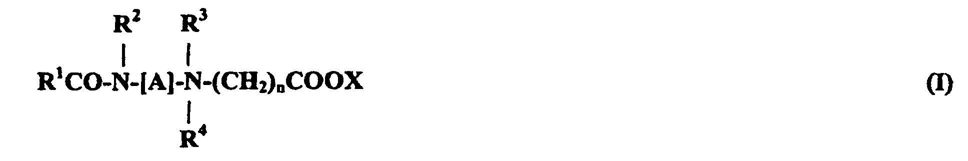 in der R¹CO für einen gesättigten und/oder ungesättigten, ethoxylierten Hydroxyacylrest mit 18 Kohlenstoffatomen sowie 1 bis 50 Oxyethyleneinheiten, A für einen linearen oder verzweigten Alkylenrest mit 1 bis 6 Kohlenstoffatomen, R², R³ und R⁴ unabhängig voneinander für Wasserstoff oder eine Alkylgruppe mit 1 bis 4 Kohlenstoffatomen, n für Zahlen von 1 bis 3 und X für Wasserstoff, ein Alkali- oder Erdalkalimetall oder Ammonium steht; bei dem man
(a) Ricinusöl und/oder gehärtetes Ricinusöl mit Diaminen der Formel **(II)** umsetzt, 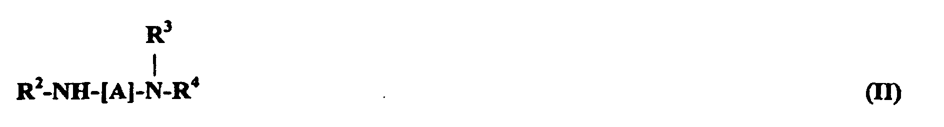 in der A für einen linearen oder verzweigten Alkylenrest mit 1 bis 6 Kohlenstoffatomen und R², R³ und R⁴ unabhängig voneinander für Wasserstoff oder eine Alkylgruppe mit 1 bis 4 Kohlenstoffatomen stehen,
(b) die resultierende Amidoamine mit Halogenalkansäuren der Formel **(III)** betainisiert,
**Hal-(CH**_{**2**}**)**_{**n**}**COOX** **(III)**
in der Hal für Halogen, n für Zahlen von 1 bis 3 und X für Wasserstoff, ein Alkali- oder Erd-alkalimetall oder Ammonium steht, und
(c) die resultierenden Betaine mit - bezogen auf die Hydroxylgruppen im Acylrest - 1 bis 50 Mol Ethylenoxid ethoxyliert.

5. Verfahren nach mindestens einem der Ansprüche 2 bis 4, **dadurch gekennzeichnet, daß** man die Ethoxylierung mit 5 bis 10 Mol Ethylenoxid - bezogen auf die Hydroxylgruppen im Acylrest - durchführt.

6. Verfahren nach mindestens einem der Ansprüche 2 bis 5, **dadurch gekennzeichnet, daß** man Dimethylaminopropylamin einsetzt.

7. Verfahren nach mindestens einem der Ansprüche 2 bis 6, **dadurch gekennzeichnet, daß** man Natriumchloracetat einsetzt.

8. Verwendung der Betaine nach Anspruch 1 zur Herstellung von kosmetischen- und/oder pharmazeutischen Zubereitungen.

## Claims

1. Betaines corresponding to formula **(I):** 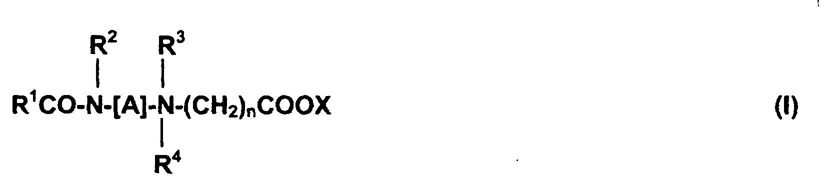 in which R¹CO is a saturated and/or unsaturated ethoxylated hydroxyacyl group containing 18 carbon atoms and 1 to 50 oxyethylene units, A is a linear or branched alkylene group containing 1 to 6 carbon atoms, R², R³ and R⁴ independently of one another represent hydrogen or an alkyl group containing 1 to 4 carbon atoms, n is a number of 1 to 3 and X is hydrogen, an alkali metal or alkaline earth metal or ammonium.

2. A process for the production of betaines corresponding to formula **(I):** 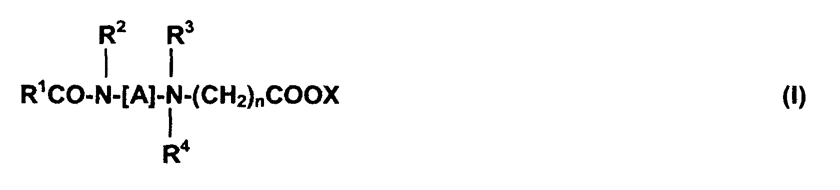 in which R¹CO is a saturated and/or unsaturated ethoxylated hydroxyacyl group containing 18 carbon atoms and 1 to 50 oxyethylene units, A is a linear or branched alkylene group containing 1 to 6 carbon atoms, R², R³ and R⁴ independently of one another represent hydrogen or an alkyl group containing 1 to 4 carbon atoms, n is a number of 1 to 3 and X is hydrogen, an alkali metal or alkaline earth metal or ammonium,
**characterized in that**
(a) castor oil and/or hydrogenated castor oil is/are ethoxylated with 1 to 50 mol - based on the hydroxyl groups in the acyl group - of ethylene oxide,
(b) the resulting ethoxylates are reacted with diamines corresponding to formula **(II):** 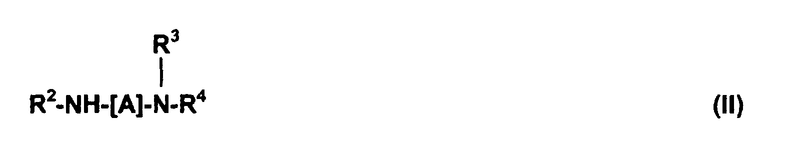 in which A is a linear or branched alkylene group containing 1 to 6 carbon atoms and R², R³ and R⁴ independently of one another represent hydrogen or an alkyl group containing 1 to 4 carbon atoms, and
(c) the resulting amidoamines are betainized with haloalkanoic acids corresponding to formula **(III):**
**Hal-(CH**_{**2**}**)**_{**n**}**COOX** **(III)**
in which Hal stands for halogen, n is a number of 1 to 3 and X is hydrogen, an alkali metal or alkaline earth metal or ammonium.

3. A process for the production of betaines corresponding to formula **(I):** 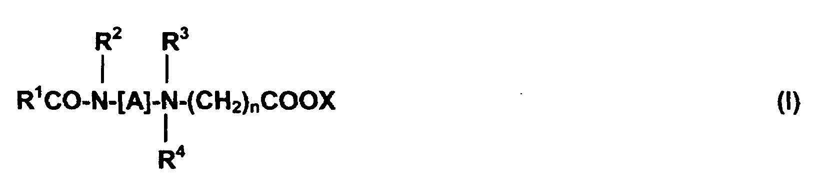 in which R¹CO is a saturated and/or unsaturated ethoxylated hydroxyacyl group containing 18 carbon atoms and 1 to 50 oxyethylene units, A is a linear or branched alkylene group containing 1 to 6 carbon atoms, R², R³ and R⁴ independently of one another represent hydrogen or an alkyl group containing 1 to 4 carbon atoms, n is a number of 1 to 3 and X is hydrogen, an alkali metal or alkaline earth metal or ammonium,
**characterized in that**
(a) castor oil and/or hydrogenated castor oil is/are reacted with diamines corresponding to formula **(II):** 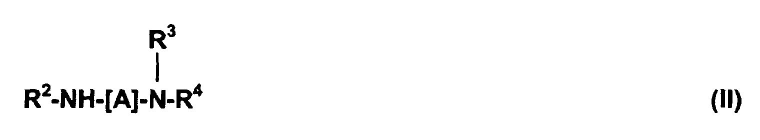 in which A is a linear or branched alkylene group containing 1 to 6 carbon atoms and R², R³ and R⁴ independently of one another represent hydrogen or an alkyl group containing 1 to 4 carbon atoms,
(b) the resulting amidoamines are ethoxylated with 1 to 50 mol - based on the hydroxyl groups in the acyl group - of ethylene oxide and
(c) the resulting ethoxylated amidoamines are betainized with haloalkanoic acids corresponding to formula **(III):**
**Hal-(CH**_{**2**}**)**_{**n**}**COOX** **(III)**
in which Hal stands for halogen, n is a number of 1 to 3 and X is hydrogen, an alkali metal or alkaline earth metal or ammonium.

4. A process for the production of betaines corresponding to formula **(I):** 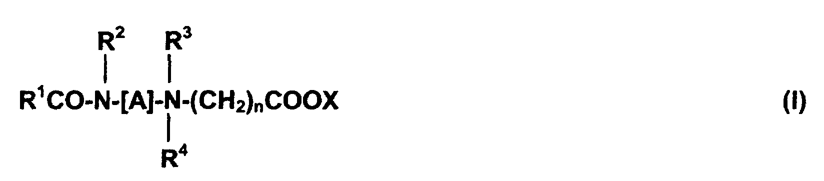 in which R¹CO is a saturated and/or unsaturated ethoxylated hydroxyacyl group containing 18 carbon atoms and 1 to 50 oxyethylene units, A is a linear or branched alkylene group containing 1 to 6 carbon atoms, R², R³ and R⁴ independently of one another represent hydrogen or an alkyl group containing 1 to 4 carbon atoms, n is a number of 1 to 3 and X is hydrogen, an alkali metal or alkaline earth metal or ammonium,
**characterized in that**
(a) castor oil and/or hydrogenated castor oil is/are reacted with diamines corresponding to formula **(II):** 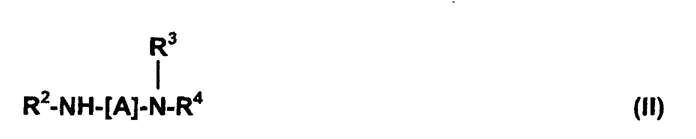 in which A is a linear or branched alkylene group containing 1 to 6 carbon atoms and R², R³ and R⁴ independently of one another represent hydrogen or an alkyl group containing 1 to 4 carbon atoms,
(b) the resulting amidoamines are betainized with haloalkanoic acids corresponding to formula **(III):**
**Hal-(CH**_{**2**}**)**_{**n**}**COOX** **(III)**
in which Hal stands for halogen, n is a number of 1 to 3 and X is hydrogen, an alkali metal or alkaline earth metal or ammonium and
(c) the resulting betaines are ethoxylated with 1 to 50 mol - based on the hydroxyl groups in the acyl group - of ethylene oxide.

5. A process as claimed in at least one of claims 2 to 4, **characterized in that** the ethoxylation is carried out with 5 to 10 mol of ethylene oxide, based on the hydroxyl groups in the acyl group.

6. A process as claimed in at least one of claims 2 to 5, **characterized in that** dimethylaminopropylamine is used.

7. A process as claimed in at least one of claims 2 to 6, **characterized in that** sodium chloroacetate is used.

8. The use of the betaines claimed in claim 1 for the production of cosmetic and/or pharmaceutical preparations.

## Revendications

1. Bétaïnes de formule (I), 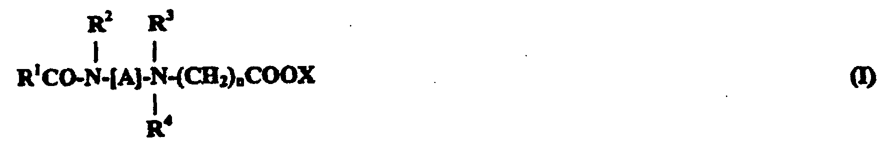 dans laquelle R¹CO représente un radical hydroxyacyle éthoxylé, saturé et/ou insaturé, ayant 18 atomes de carbone et comportant de 1 à 50 unités oxyéthylène, A représente un radical alkylène linéaire ou ramifié ayant de 1 à 6 atomes de carbone, R¹, R³ et R⁴ représentent, indépendamment les uns des autres, un atome d'hydrogène ou un groupe alkyle ayant de 1 à 4 atomes de carbone, n représente des nombres de 1 à 3, et X représente un atome d'hydrogène, un métal alcalin ou alcalino-terreux ou l'ammonium.

2. Procédé pour la préparation de bétaïnes de formule (I), 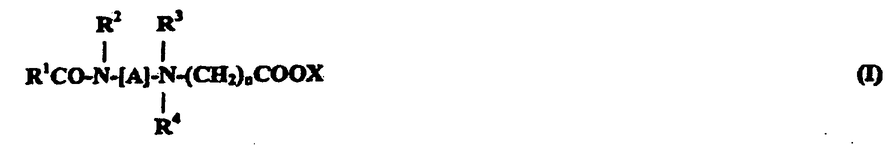 dans laquelle R¹CO représente un radical hydroxyacyle éthoxylé, saturé et/ou insaturé, ayant 18 atomes de carbone et comportant de 1 à 50 unités oxyéthylène, A représente un radical alkylène linéaire ou ramifié ayant de 1 à 6 atomes de carbone, R¹, R³ et R⁴ représentent, indépendamment les uns des autres, un atome d'hydrogène ou un groupe alkyle ayant de 1 à 4 atomes de carbone, n représente des nombres de 1 à 3, et X représente un atome d'hydrogène, un métal alcalin ou alcalino-terreux ou l'ammonium, selon lequel
(a) on soumet à une éthoxylation de l'huile de ricin et/ou de l'huile de ricin durcie, avec - par rapport aux groupes hydroxy dans le radical acyle - de 1 à 50 moles d'oxyde d'éthylène,
(b) on fait réagir les produits d'éthoxylation résultants avec des diamines de formule (II), 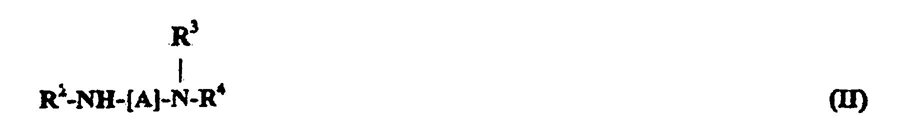 dans laquelle A représente un radical alkylène linéaire ou ramifié ayant de 1 à 6 atomes de carbone et R², R³ et R⁴ représentent, indépendamment les uns des autres, un atome d'hydrogène ou un groupe alkyle ayant de 1 à 4 atomes de carbone, et
(c) on transforme en bétaïnes les amidoamines résultantes, avec des acides halogénoalcanoïques de formule (III),
Hal-(CH₂)ₙCOOX (III)
dans laquelle Hal représente un atome d'halogène, n représente des nombres allant de 1 à 3, et X représente un atome d'hydrogène, un métal alcalin ou alcalino-terreux ou l'ammonium.

3. Procédé pour la préparation de bétaïnes de formule (I), 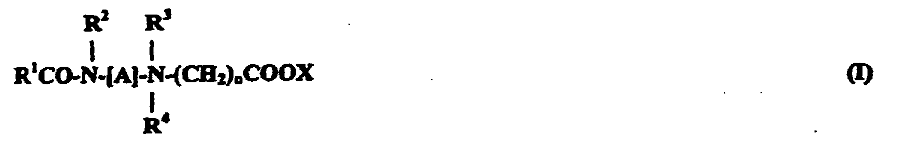 dans laquelle R¹CO représente un radical hydroxyacyle éthoxylé, saturé et/ou insaturé, ayant 18 atomes de carbone et comportant de 1 à 50 unités oxyéthylène, A représente un radical alkylène linéaire ou ramifié ayant de 1 à 6 atomes de carbone, R¹, R³ et R⁴ représentent, indépendamment les uns des autres, un atome d'hydrogène ou un groupe alkyle ayant de 1 à 4 atomes de carbone, n représente des nombres de 1 à 3, et X représente un atome d'hydrogène, un métal alcalin ou alcalino-terreux ou l'ammonium,
selon lequel
(a) on fait réagir de l'huile de ricin et/ou de l'huile de ricin durcie, avec des diamines de formule (II), 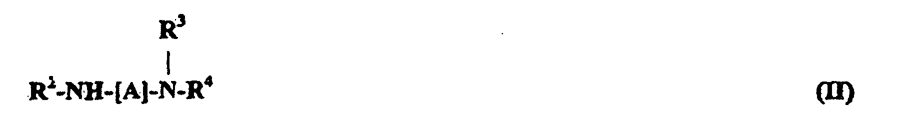 dans laquelle A représente un radical alkylène linéaire ou ramifié ayant de 1 à 6 atomes de carbone et R², R³ et R⁴ représentent, indépendamment les uns des autres, un atome d'hydrogène ou un groupe alkyle ayant de 1 à 4 atomes de carbone,
(b) on soumet les amidoamines résultantes à une éthoxylation avec - par rapport aux groupes hydroxy dans le radical acyle - de 1 à 50 moles d'oxyde d'éthylène, et
(c) on transforme en bétaïnes les amidoamines éthoxylées résultantes, avec des acides halogénoalcanoïques de formule (III),
Hal-(CH₂)ₙCOOX (III)
dans laquelle Hal représente un atome d'halogène, n représente des nombres de 1 à 3, et X représente un atome d'hydrogène, un métal alcalin ou alcalino-terreux ou l'ammonium

4. Procédé pour la préparation de bétaïnes de formule (I), 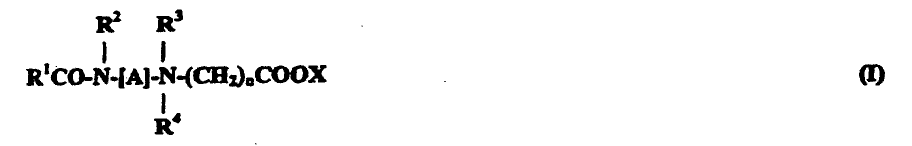 dans laquelle R¹CO représente un radical hydroxyacyle éthoxylé, saturé et/ou insaturé, ayant 18 atomes de carbone et comportant de 1 à 50 unités oxyéthylène, A représente un radical alkylène linéaire ou ramifié ayant de 1 à 6 atomes de carbone, R¹, R³ et R⁴ représentent, indépendamment les uns des autres, un atome d'hydrogène ou un groupe alkyle ayant de 1 à 4 atomes de carbone, n représente des nombres de 1 à 3, et X représente un atome d'hydrogène, un métal alcalin ou alcalino-terreux ou l'ammonium,
selon lequel
(a) on fait réagir de l'huile de ricin et/ou de l'huile de ricin durcie, avec des diamines de formule (II), 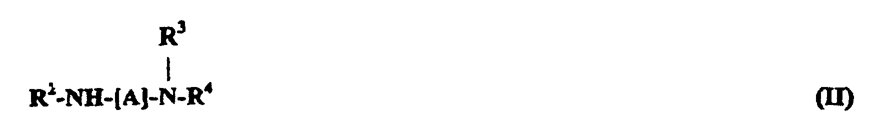 dans laquelle A représente un radical alkylène linéaire ou ramifié ayant de 1 à 6 atomes de carbone et R², R³ et R⁴ représentent, indépendamment les uns des autres, un atome d'hydrogène ou un groupe alkyle ayant de 1 à 4 atomes de carbone,
(b) on transforme en bétaïnes les amidoamines résultantes, avec des acides halogénoalcanoïques de formule (III),
Hal-(CH₂)ₙCOOX (III)
dans laquelle Hal représente un atome d'halogène, n représente des nombres de 1 à 3, et X représente un atome d'hydrogène, un métal alcalin ou alcalino-terreux ou l'ammonium, et
(c) on soumet à une éthoxylation les bétaïnes résultantes, avec - par rapport aux groupes hydroxy dans le radical acyle - 1 à 50 moles d'oxyde d'éthylène.

5. Procédé selon au moins l'une des revendications 2 à 4,
**caractérisé en ce qu'**
on effectue l'éthoxylation avec 5 à 10 moles d'oxyde d'éthylène - par rapport aux groupes hydroxy dans le radical acyle.

6. Procédé selon au moins l'une des revendications 2 à 5,
**caractérisé en ce qu'**
on utilise de la diméthylaminopropylamine.

7. Procédé selon au moins l'une des revendications 2 à 6,
**caractérisé en ce qu'**
on utilise du chloracétate de sodium.

8. Utilisation des bétaïnes selon la revendication 1,
pour la préparation de préparations cosmétiques et/ou pharmaceutiques.
